# EUROPEAN PATENT APPLICATION

(11) **EP 4 450 071 A1**
(43) Date of publication of application: **23.10.2024**
(21) Application number: 23169023.1
(22) Date of filing: 20.04.2023
(51) Int. Cl.: A61K 31/4415, A61K 31/4745, A61K 31/513, A61K 31/519, A61K 31/555, A61P 35/00

(54) **[6R]-MTHF IN B6 ENHANCED 5-FU BASED CHEMOTHERAPY OF PANCREATIC CANCER**

(71) Applicant: Isofol Medical AB, 413 46 Göteborg (SE)
(72) Inventor: LINDBERG, Per Lennart, 413 01 Gothenburg (SE); MACHOVER, David, 75005 Paris (FR)
(74) Representative: AWA Denmark A/S

(57) **Abstract**

The present invention relates to the treatment of pancreatic cancer, in particular KRAS-mutated pancreatic adenocarcinoma, in human populations employing a dosage regimen which includes arfolitixorin ([6R]-5,10-methylenetetrahydrofolate ([6R]-MTHF) and a B6 vitamin in connection with 5-fluorouracil (5-FU) based chemotherapy.

## Description

### BACKGROUND OF THE INVENTION

Pancreatic cancer is the fourth leading cause of cancer death in the United States². The incidence of pancreatic cancer is highest in Europe (7.7 per 100,000 people) and North America (7.6 per 100,000 people), followed by Oceania (6.4 per 100,000 people)³. It is estimated that in 2021 in the United States, there will be 60,430 new cases and 48,220 deaths related to pancreatic cancer⁴.

Pancreatic ductal adenocarcinoma (PDAC) is an exocrine pancreatic cancer and the most common type of pancreatic cancer with a dismal 5-year overall survival rate of 10%⁵. Patients with metastatic PDAC (mPDAC) at diagnosis have a 5-year overall survival rate of only 3%, a number which has not changed significantly over the last two decades, despite some advances in therapy and pharmacological therapy. Surgical resection is the only curative modality with a 5-year survival rate (after resection) of ~20%. However, only ≤20% of PDAC patients are considered surgical candidates, as the majority of patients present with advanced disease at diagnosis. In time, most of these locally advanced cancers will also metastasize⁶.

Consequently, there is a strong medical need for a more effective treatment of mPDAC. Until 2011, the standard first-line treatment for metastatic pancreatic cancer was gemcitabine, which yielded a median overall survival (mOS) of about 6 months. Introduction of the FOLFIRINOX regimen (5-fluorouracil [5-FU] in combination with leucovorin, irinotecan and oxaliplatin) yielded an objective response rate (ORR) of 31.6% and mOS of 11.1 months compared to gemcitabine monotherapy with an ORR of 9.4% and mOS of 6.8 months in the randomized study PRODIGE 4 with 342 patients enrolled'. In 2013, the MPACT study comparing gemcitabine plus nab-paclitaxel to gemcitabine also demonstrated superior activity of the combination with an ORR of 23% and mOS of 8.5 months relative to gemcitabine monotherapy with an ORR 7% and mOS 6.7 months⁸.

The current standard of care for first-line treatment of locally advanced unresectable or mPDAC by the National Comprehensive Cancer Network and the European Society of Medical Oncology based on evidence from large phase 3 trials is chemotherapy with FOLFIRINOX⁷⁻¹⁰. FOLFIRINOX is a combination of irinotecan, oxaliplatin, 5-FU and leucovorin. Irinotecan hydrochloride is an antineoplastic agent whose active metabolite, SN38, inhibits topoisomerase I and produces deoxyribonucleic acid (DNA) breakage and cell death. Oxaliplatin is a platinum-based drug that forms crosslinks of guanine that inhibit DNA replication and transcription. 5-FU is an antineoplastic antimetabolite that interferes with the synthesis of DNA (and to a lesser extent ribonucleic acid [RNA]) by inhibiting the enzyme thymidylate synthase (TS), thus interrupting supply of thymidine for DNA synthesis in the presence of leucovorin, a reduced folate. As defined in the phase 3 study ACCORD 11/0402, the FOLFIRINOX regimen consists of a 400 mg bolus of 5-FU followed by 2400 mg/m² 5-FU infused i.v. over 46 hours, 400 mg/m² leucovorin i.v. infusion, 85 mg/m² oxaliplatin i.v. infusion, and 180 mg/m² irinotecan i.v. infusion. Based on phase 2 trials, other variations of FOLFIRINOX have been recognized as acceptable alternatives that retain comparable clinical efficacy with an improved side effect profile¹¹⁻¹⁴. The response rate associated with administration of the FOLFIRINOX regimen is 31.6%⁷.

However, triplet regimens such as FOLFIRINOX are in general associated with a highly increased level of adverse side effects. In a meta-analysis of patient data from 5 clinical studies it was thus found that - compared with patients receiving doublet therapy (FOLFOX or FOLFIRI) - patients in the triplet group (FOLFIRINOX plus bevacizumab) had higher rates of grade 3 or 4 neutropenia (45.8% vs 21.5%, P < .001), febrile neutropenia (6.3% vs 3.7%, P = .019), nausea (5.5% vs 3.0%, P = .016), mucositis (5.1% vs 2.9%, P = .024), and diarrhea (17.8% vs 8.4%, P < .001). Death due to toxicity occurred in 2.3% vs 1.4% of patients (P = .277) (Cremolini 2020).

Experiments conducted in the human colon carcinoma HT29 and HCT116 cell lines, and in the murine leukemia L1210 cell line *in vitro* to investigate for interactions between 5-FU, leucovorin and the cofactor pyridoxal 5'-phosphate (PLP) on cell growth has shown that supplementation of cancer cells exposed to 5-FU with high concentrations of PLP and leucovorin potentiated the cytotoxic activity of 5-FU in the three cell lines (Machover 2018). In a later study (Machover 2021) five patients with advanced metastatic colorectal adenocarcinoma were treated according to the classical FOLFIRINOX triplet regimen (leucovorin, 5-FU, irinotecan, and oxaliplatin) supplemented with pyridoxine in high doses.

Antitumor activity was found to be high for several patients, but due to the small scale of the study, lack of controls and varied patient disease history it was not clear whether the co-administration of pyridoxine led to a significantly different result from what would have been expected from employing standard-of-care treatment on the same group of patients. Assessment of toxicity associated with the pyridoxine-modified protocols was however of the same magnitude as expected for the standard protocols.

Triplet regimens therefore remains recommended only for patients whose general condition is very good (ECOG 0), estimated to less than 40% of all metastatic pancreatic patients (e.g. Hang 2018). For the majority of such patients, there thus remains an unmet need for an improved folate/fluoropyrimidine-based treatment protocol of their disease, such as a more efficient and better tolerated triplet chemotherapeutic regimen.

### DEFINITIONS

As used herein, the term **Leucovorin^{®}** or **folinic acid** shall both mean 5-formyl tetrahydrofolic acid, i.e. the 5-formyl derivative of tetrahydrofolic acid. Folinic acid contains 2 asymmetric centers. **Leucovorin^{®}** (LV) is composed of a 1:1 mixture of the two dextrorotary and levorotary diastereomers (*d*-leucovorin (*d*-LV, (6R,2'S)-configuration) and *l*-leucovorin (*l*-LV, (6S,2'S)-configuration), respectively), and may also be referred to as **(*d,l*-LV).**

As used herein, the term **Levoleucovorin** shall refer to the commercially available product which contains only the natural and pharmacologically active *levo*-isomer *l*-LV (or LLV). In vitro, *l*-LV has been shown to be rapidly converted to the biologically available methyl-tetrahydrofolate form while the *dextro* form *d*-LV (DLV) is slowly excreted by the kidneys. Leucovorin and levoleucovorin have however been shown to be pharmacokinetically identical and may be used interchangeably with limited differences in efficacy (considering the inactive d-isomer in LV) or side effects (see Kovoor et al, Clin Colorectal Cancer 8 200-6 (2009).

As used herein, the terms **MTHF** or **methyleneTHF** shall both refer to 5,10-Methylene-5,6,7,8-tetrahydrofolate.

As used herein, the terms **racemic methyleneTHF, CoFactor^{®}** or **[6R,S]-5,10-methyleneTHF** shall all refer to the 1:1 diastereomeric mixture [6R,S]-5,10-Methylene-5,6,7,8-tetrahydrofolate.

As used herein, the terms arfolitixorin and [6R]-5,10-MTHF shall both refer to the single diastereomer, [6R]-5,10-methylenetetrahydrofolate.

As used herein, the terms IV or i.v. shall both mean **intravenous.**

As used herein, the term **ORR** shall refer to the Objective Response Rate, i.e. the proportion of patients with a reduction in tumor burden of a predefined amount. This shall be calculated as follows: ORR = Sum of partial responses plus complete responses as per RECIST 1.1 (a set of published rules (link: https://recist.eortc.org/recist-1-1-2/) that define when tumors in cancer patients progress during treatments, the responses being defined as:
**Complete Response (CR):**
   - Disappearance of all target lesions. Any pathological lymph nodes (whether target or non-target) must have reduction in short axis to <10 mm.
**Partial Response (PR):**
   - At least a 30% decrease in the sum of diameters of target lesions, taking as reference the baseline sum diameters.
**Progressive Disease (PD):**
   - At least a 20% increase in the sum of diameters of target lesions, taking as reference the smallest sum on study (this includes the baseline sum if that is the smallest on study).
   - In addition to the relative increase of 20%, the sum must also demonstrate an absolute increase of at least 5 mm. (Note: the appearance of one or more new lesions is also considered progression).
**Stable Disease (SD):**
   - Neither sufficient shrinkage to qualify for PR nor sufficient increase to qualify for PD, taking as reference the smallest sum diameters while on study.

As used herein, the term **BSA** refers to Body Surface Area

**FOLFOXIRI** and **FOLFIRINOX** are chemotherapy regimens consisting of leucovorin, fluorouracil, irinotecan, and oxaliplatin.

**FOLFIRI** is a chemotherapy regimen consisting of leucovorin, fluorouracil, and irinotecan.

**FOLFOX** is a chemotherapy regimen consisting of leucovorin, fluorouracil, and oxaliplatin.

**B6** vitamins are a group of compounds that encompasses six interconvertible compounds (i.e., B6 vitamers), namely pyridoxine (PN); pyridoxamine (PM); pyridoxal (PL); and their respective 5'-phosphorylated forms, pyridoxine 5'-phosphate (PNP), pyridoxamine 5'-phosphate (PMP), and the cofactor pyridoxal 5'-phosphate (PLP).

### SUMMARY OF INVENTION

Arfolitixorin (Modufolin^{®}) is a new drug developed to increase the efficacy of the cytotoxic agent 5-fluorouracil (5-FU) and as a rescue drug after high-dose methotrexate treatment. Arfolitixorin, [6R]-5,10-methylenetetrahydrofolate, abbreviated herein as [6R]-5,10-MTHF, needs to be metabolically formed when using the widely used folate-based drugs leucovorin and levoleucovorin. Arfolitixorin, however, does not require metabolic activation to exert its effect and may therefore be suitable for all patients.

Applicant's previous work relating to "doublet" chemotherapeutic regimens (arfolitixorin plus 5-FU + either oxaliplatin or iriniotecan) has been described in i.a. WO 2019/037898 and WO 2019/037899.

Applicant has also investigated the more intense "triplet" chemotherapeutic regimens with a view to develop a more efficient and/or better tolerated folate-based regimen than the current state-of-the art FOLFOXIRI/FOLFIRINOX regimens (which employ leucovorin as the folate) comprising arfolitixorin, 5-FU, irinotecan and oxaliplatin.

These investigations have led to the surprising finding that previously untreated patients diagnosed with RAS-mutated pancreatic adenocarcinoma may be treated according to a B6 vitamin-enhanced triplet chemotherapeutic protocol involving administration of:
- 5-fluorouracil, irinotecan, and oxaliplatin
- arfolitixorin ([6R]-5,10-MTHF)
- A B6 vitamin selected from pyridoxamine (PM) or pyridoxine (PN)
by which treatment best ORRs (objective response rates) of >50% can be achieved, and which treatment induces fewer side effects and is thus better tolerated than standard-of-care FOLFOXIRI/FOLFIRINOX regimens.

Accordingly, in a **first aspect** of the invention, arfolitixorin is provided for use in a human patient in the treatment of *KRAS*-mutant pancreatic adenocarcinoma, which treatment comprises the following steps:
**On Day 1**
   a) administering a continuous IV infusion over 120 min ± 5 min containing 80 mg/m² oxaliplatin, followed by
   b) administering a continuous IV infusion over 30 min ± 3 min containing 180 ±5 mg/m² irinotecan, followed by
   c) administering a continuous IV infusion over 30 min ± 3 min containing 120 - 400 mg/m² arfolitixorin, followed by
   d) administering an IV infusion over 30 min ± 3 min containing 3000 - 6000 mg pyridoxine, followed by
   e) administering a continuous IV infusion over 120 min ± 10 min containing 625 ±5 mg/m² 5-fluorouracil (5-FU), followed by
   f) administering a continuous IV infusion containing 375 ±5 mg/m² 5-fluorouracil over 22 ± 1 hour, followed by
**On Day 2**
   g) administering a continuous IV infusion over 30 min ± 3 min containing 120 - 400 mg/m² arfolitixorin, followed by
   h) administering an IV infusion over 30 min ± 3 min containing 3000 - 6000 mg pyridoxine, followed by
   i) administering a continuous IV infusion over 120 min ± 10 min containing 625 ±5 mg/m² 5-FU, followed by
   j) administering a continuous IV infusion containing 375 mg/m² 5-FU over 22 ± 1 hour,
wherein said human patient has been found by genotype testing to have *KRAS*-mutant pancreatic adenocarcinoma, and wherein all steps a) - j) are repeated every 2 weeks for a total treatment period of at least 16 weeks or until termination of the treatment.

A treatment course comprises a two-day protocol as outlined above, which course is then repeated every 14 days. Assessment of antitumor efficacy is performed after each phase of 4 courses, i.e., before the 5^{th} course, before the 9^{th} course, etc.

The treatment based on the protocol according to the first aspect of the present invention may in principle be terminated "for any reason", such as e.g. by a patient decision or a decision taken by the responsible medical person, i.a. due to disease progression or adverse events. Furthermore, the protocols may be interrupted by treatment holidays and the like. Finally the responsible medical person may decide on a fixed number of treatment cycles.

### DESCRIPTION OF FIGURES

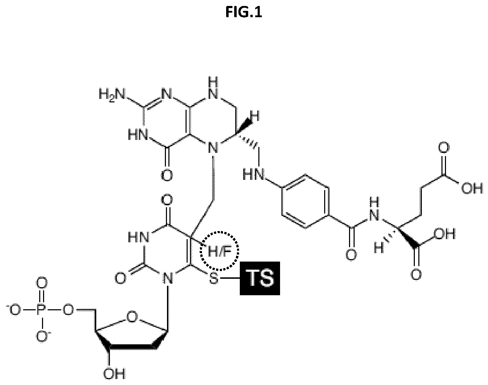

**Fig. 1****.** Ternary complex formed between [6R]-5,10-methylene-THF, thymidylate synthase, and 2'-deoxy-uridine-5'-monophosphate, 2-dUMP or 5-fluoro-2'-deoxy-uridine-5'-monophosphate, 5-FdUMP. The dotted circle marks the difference between 2-dUMP and 5-FdUMP. THF: tetrahydrofolate; TS: thymidylate synthase. (Adapted from Danenberg 2016)

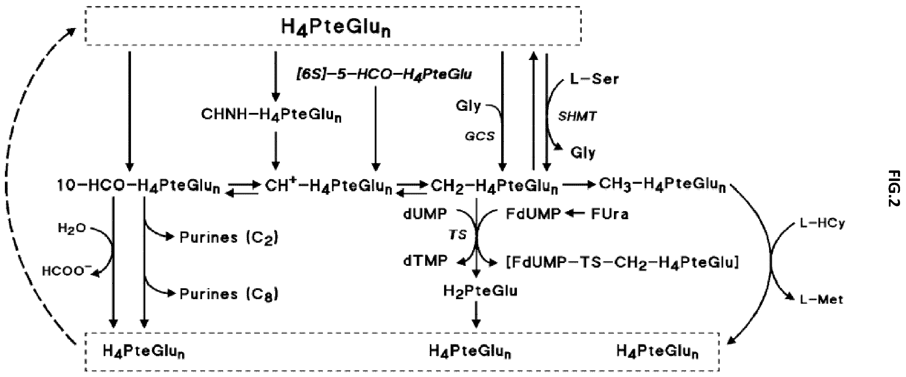

**Fig. 2**. Selected pathways of folates and FdUMP-mediated thymidylate synthase (TS) inhibition. *Folates:* **H2PteGlu:** 7,8-dihydro pteroylglutamate; **H4PteGlu:** 5,6,7,8- tetrahydro pteroylglutamate; **CH2-H4PteGlu:** 5,10-methylene tetrahydro pteroylglutamate; **CH3-H4PteGlu:** 5-methyl tetrahydro pteroylglutamate; **CH⁺-H4PteGlu:** 5,10-methenyl tetrahydropteroylglutamate; **10-HCO-H4PteGlu:** 10-formyl tetrahydropteroylglutamate; **CHNH-H4PteGlu:** 5-formimino tetra hydro pteroylglutamate; **[6S]-5-HCO-H4PteGlu:** [6S]-5-formyl tetrahydro pteroylglutamate ([6S]-folinic acid). *Enzymes:* **TS,** thymidylate synthase; **SHMT,** serine hydroxymethyltransferase (pyridoxal 5'-phosphate-dependent enzyme, including the cytoplasmic SHMT1 and the mitochondrial SHMT2 isoforms); **GCS,** glycine cleavage system (mitochondrion). *Other compounds and substances involved in TS inhibition:* **dUMP,** deoxy uridine monophosphate; **dTMP,** thymidine monophosphate; **L-Ser,** L-serine; **Gly,** glycine; **L-HCy,** L-homocysteine; **L-Met,** L-methionine; **HCOO⁻,** formate; **FUra,** 5-fluorouracil (5-FU); **FdUMP,** fluorodeoxyuridine monophosphate; **[FdUMP-TS-CH2-H4PteGlu],** the ternary complex resulting in inhibition of TS (see also fig.1). Synthesis of methylene tetrahydrofolate from THF results mainly from the transfer of Cβ of serine to THF catalyzed by SHMT, which is a ubiquitous pyridoxal 5'-phosphate (PLP)-dependent enzyme that is the major source of one-carbon units for cellular metabolism. (Figure Adapted from Machover 2021)

### DETAILED DESCRIPTION OF THE INVENTION

In 5-FU-based cancer treatment, 5-FU is converted to its active metabolite 5-fluoro-2'-deoxyuridine 5'-monophosphate (FdUMP), which causes cytotoxicity by inhibiting thymidylate synthase (TS), the key enzyme involved in the normal natural methylation of dUMP to dTMP, which is necessary for DNA replication and repair through the synthesis of nucleotides. The inhibition of TS, taking place via the formation of an inhibitory ternary complex between FdUMP, TS and the co-substrate [6R]-5,10-methylenetetrahydrofolic acid ([6R]-MTHF), leads to suppression of DNA synthesis and cell death by apoptosis¹⁷. However, since the binding of [6R]-MTHF to the ternary complex is reversible, increased concentration of this molecule will stabilize the ternary complex, providing a more pronounced inhibition of TS.

For many years now, the diasteromeric mixture leucovorin (LV) (i.e., mixture of [6S]- and [6R]-5-formyl-THF), and more recently levoleucovorin (I-LV, [6S]-5-formyl-THF only), have been used for increased efficacy in cancer therapy in combination with 5-FU. Thus, both LV and I-LV have been used therapeutically as the reduced folate source for metabolically supplying the other natural reduced folate ([6R]-MTHF), required to stabilize the inhibitory ternary complex. The synergy with 5-FU was early found to improve the efficacy by doubling the ORR.

Arfolitixorin can now be used in combination with 5-fluorouracil (5-FU) to enhance 5-FU inhibition of thymidylate synthase (TS). As above, 5-FU undergoes metabolic transformation to 5-fluorodeoxyuridine monophosphate (FdUMP). TS interacts with FdUMP and arfolitixorin, now being the active [6R]-MTHF itself, interacts to form the "inhibitory" ternary complex. The resulting inhibition of TS reduces the supply of thymidine for DNA synthesis resulting in the death of proliferating tumor cells.

However, the binding of arfolitixorin to this ternary complex can be reversed due to insufficient [6R]-MTHF and therefore the stability of the ternary complex is dependent on the concentration of arfolitixorin, i.e., the pharmaceutical entity itself. The higher the cellular concentration of arfolitixorin, the higher stability of the ternary complex and the greater the inhibition of TS.

Arfolitixorin has been in development for a number of years and has been studied in several clinical studies. During one of these studies (the Phase I/IIa study ISO-CC-005) it was surprisingly discovered in December 2017 that administration of [6R]-MTHF (arfolitixorin) and 5-FU according to a particular treatment regimen over a treatment period of at least 8 weeks lead to a prevention or retarding of the progression in a human of solid tumors. No statistically significant progression of said solid tumors was observed between 8 and 16 weeks after initiating treatment. These results are discussed *i.a.* in applicant's international patent application WO 2019/037898 and WO 2019/037899.

The completion of the study was announced in January 2020. In total, 105 colorectal cancer patients were included in the study. The data showed a best overall response rate (ORR) of 55 %. These patients had been treated with the "doublet" regimens comprising 120 mg/m² arfolitixorin and 5-fluorouracil (5-FU) with either irinotecan or oxaliplatin (ARFIRI/ARFOX).

In addition to the doublet folate-based chemotherapeutic regimens (ARFIRI/ARFOX) for the treatment of colorectal cancer, applicant has also been interested in developing improved versions of the more intensive "triplet regimens", such as the classical FOLFOXIRI/FOLFIRINOX (which employ leucovorin as the folate), i.e. versions comprising arfolitixorin, 5-FU, irinotecan and oxaliplatin, since on the one hand many patients with advanced metastatic CRC and other aggressive cancer forms like pancreatic adenocarcinoma would benefit from such a high-intensity treatment whilst on the other hand a large proportion of such patients would not be eligible to high-intensity treatment due to the accompanying side-effects associated with the classical triplet strategies.

Leucovorin and I-leucovorin, both currently approved for treatment of gastrointestinal and other cancers, need to be metabolically activated to [6R]-MTHF to potentiate the effect of 5-FU. This dependency on cellular activation provides a hurdle for reaching sufficient levels of the active [6R]-MTHF. Arfolitixorin treatment has been shown to result in [6R]-MTHF levels that are at least 3-4 times higher than with comparable treatment with leucovorin, and consequently generates the preconditions for a substantial increase in the efficacy of 5-FU. Secondly, the enzymatic activation is genetically regulated^{16,18}, and is therefore associated with variability between individuals and conditions. Arfolitoxirin is believed to be efficacious in a larger proportion of patients with less inter- and intra-individual variability. These differences may potentially turn out to be of significant importance for increased clinical efficacy in 5-FU based cancer treatment.

Applicant thus speculated that by augmenting the effect of the folate component in the classical triplet regimen by replacement of leucovorin with arfolitixorin, it might be possible to develop a better tolerated, and potentially also more efficient triplet chemotherapeutic regimen for the treatment of aggressive cancer forms, such as pancreatic adenocarcinoma with KRAS mutations.

In the treatment of cancer with 5-fluorouracil, the administration of folates mechanistically leads to the formation of [6R]-5,10-methylene-tetrahydrofolate, and the increased concentration of this molecule leads to stabilization of the "ternary complex" comprising thymidylate synthase (TS), 2'-deoxy-uridine-5'-monophosphate (dUMP), and [6R]-5,10-methylene-tetrahydrofolate (Danenberg 2016).

[6R]-5,10-methylene-THF is the key endogenous one-carbon donor and a co-substrate of the TS enzyme for methylation of dUMP to 2'-deoxy-thymidine-5'-monophosphate (dTMP). This product is one of the necessary nucleotide substrates for DNA synthesis and repair, and is thus essential for cell division and life. 5-FU is known to exert its cytotoxic activity, to a major extent, through inhibition of the TS enzyme. Importantly, for either synthesis of dTMP, via TS catalysis, or inhibition of TS, by 5-FU, a ternary complex is formed between TS, [6R]-5,10-methylene-THF, and the respective third component, either dUMP or 5-fluoro-2'-deoxy-uridine-5'-monophosphate (5-FdUMP; see Fig. 1).

Folates belong to the group of Vitamin B micronutrients (Franco 2022) which are essential regulators of the so-called one-carbon metabolism. The one-carbon metabolism is a collection of cyclical metabolic pathways that orchestrates several metabolic processes which are required for the maintenance of intracellular DNA synthesis and methylation. The one carbon pathway is comprised of the folate cycle and the methionine cycle (see Fig.2) in which in particular folate, vitamin B12 and B6 play a role. Folic acid (pteroyl-I-glutamic acid) is an oxidized folate also known as vitamin B9 which is not active in humans, but is converted in the liver to the biologically active 7,8-dihydrofolate (DHF). Folic acid does not by itself increase the concentration of [6R]-5,10-methylene-THF enough to enhance FdUMP-mediated inhibition of TS, and is thus unable to modulate 5-FU cytotoxicity at physiological concentrations.

Whereas vitamin B12 (cyanocobalamin) and folate have been reported to interact in certain situations, vitamin B6 and folate are not known to interact directly when ingested (Dierkes 1998, Shane 2008). Other observational studies of dietary or dietary plus supplementary intake of vitamin B6 and colorectal cancer risk have been inconsistent, with most studies reporting nonsignificant positive or inverse associations (Zhang 2013).

Experiments conducted in the human colon carcinoma HT29 and HCT116 cell lines, and in the murine leukemia L1210 cell line *in vitro* to investigate for interactions between 5-FU, leucovorin and the cofactor pyridoxal 5'-phosphate (PLP) on cell growth however showed that supplementation of cancer cells exposed to 5-FU with high concentrations of PLP and leucovorin strongly potentiated the cytotoxic activity of 5-FU in the three cell lines (Machover 2018).

In a later study (Machover 2021) five patients (3M, 2F) with unresectable pancreas adenocarcinoma were treated according to the classical FOLFIRINOX triplet regimen (leucovorin, 5-FU, irinotecan, and oxaliplatin) supplemented with pyridoxine in high doses accompanying each administration of leucovorin plus 5-FU. The five patients had an average ECOG score of >3. Mutation status was not stated.

Antitumor activity was found to be high for several patients, but due to the small scale of the study, lack of controls and varied disease history it is not clear whether the co-administration of pyridoxine has led to a significantly different result from what would have been expected from employing the standard FOLFIRINOX protocol on the same group of patients. Assessment of toxicity associated with the pyridoxine-modified protocols was however of the same magnitude than expected for the standard protocols.

According to the present invention, it has surprisingly been found that previously untreated patients diagnosed with RAS-mutated pancreatic adenocarcinoma may be treated according to a B6 vitamin-enhanced triplet chemotherapeutic protocol involving administration of:
- 5-fluorouracil, irinotecan, and oxaliplatin
- arfolitixorin ([6R]-5,10-MTHF)
- A B6 vitamin selected from pyridoxamine (PM) or pyridoxine (PN) by which treatment best ORRs (objective response rates) of >50% can be achieved, and which treatment induces fewer side effects and is thus better tolerated than standard-of-care FOLFOXIRI/FOLFIRINOX regimens.

Accordingly, in a **first aspect** of the invention, arfolitixorin is provided for use in a human patient in the treatment of *KRAS*-mutant pancreatic adenocarcinoma, which treatment comprises the following steps:
**On Day 1**
   a) administering a continuous IV infusion over 120 min ± 5 min containing 80 mg/m² oxaliplatin, followed by
   b) administering a continuous IV infusion over 30 min ± 3 min containing 180 ±5 mg/m² irinotecan, followed by
   c) administering a continuous IV infusion over 30 min ± 3 min containing 120 - 400 mg/m² arfolitixorin, followed by
   d) administering an IV infusion over 30 min ± 3 min containing 3000 - 6000 mg pyridoxine, followed by
   e) administering a continuous IV infusion over 120 min ± 10 min containing 625 ±5 mg/m² 5-fluorouracil (5-FU), followed by
   f) administering a continuous IV infusion containing 375 ±5 mg/m² 5-fluorouracil over 22 ± 1 hour, followed by
**On Day 2**
   g) administering a continuous IV infusion over 30 min ± 3 min containing 120 - 400 mg/m² arfolitixorin, followed by
   h) administering an IV infusion over 30 min ± 3 min containing 3000 - 6000 mg pyridoxine, followed by
   i) administering a continuous IV infusion over 120 min ± 10 min containing 625 ±5 mg/m² 5-FU, followed by
   j) administering a continuous IV infusion containing 375 mg/m² 5-FU over 22 ± 1 hour,
   wherein said human patient has been found by genotype testing to have KRAS-mutant pancreatic adenocarcinoma, and wherein all steps a) - j) are repeated every 2 weeks for a total treatment period of at least 16 weeks or until termination of the treatment.

A treatment course comprises a two-day protocol as outlined above, which course is then repeated every 14 days. Assessment of antitumor efficacy is performed after each phase of 4 courses, i.e., before the 5^{th} course, before the 9^{th} course, etc.

Throughout the present application the treatment regimen according to the first aspect is referred to as the "ARFOXIRI" protocol. The treatment based on the ARFOXIRI protocol may in principle be terminated "for any reason", such as e.g. by a patient decision or a decision taken by the responsible medical person, i.a. due to disease progression or adverse events. Furthermore, the protocol may be interrupted by treatment holidays and the like. Finally the responsible medical person may decide on a fixed number of treatment cycles.

In an embodiment, the human patient is previously untreated. In yet another embodiment, the total treatment period is at least 20 weeks, such as at least 24 weeks.

In pancreatic cancer patients the *KRAS* mutation status is traditionally determined by tumor sample analysis. This requires surgery, and the subsequent analysis (extraction of genomic DNA from the tumor biopsy and analysis for mutations using dPCR) often takes weeks to complete. This creates problems in clinical situations which require urgent treatment based on the mutation status of the patient.

However, several studies the past 5-10 years have demonstrated that genotype testing by analysis of circulating, cell-free tumor DNA (ctDNA) in plasma or serum samples is becoming increasingly accurate and thus important as a non-invasive and fast alternative or supplement to tumor sample analysis. The method is also referred to as "Liquid Biopsy" analysis. Cell-free DNA (cfDNA) is fragmented DNA that is found in the non-cellular blood components.

Analysis of ctDNA ("liquid biopsy" analysis ) is thus deemed an important tool for determining the relevant patient group according to the first or second aspect of the present invention.

Accordingly, in embodiments of the invention, arfolitixorin is provided for use in a human patient in the treatment of pancreatic adenocarcinoma, which treatment comprises steps a) -j) according to the first aspect of the invention, wherein the human patient has been found either by traditional tumor tissue analysis or by ctDNA analysis to have either *RAS* mutation-positive, such as *KRAS* mutated, or *RAS* wildtype (wt) metastatic colorectal cancer.

In preferred embodiments of any of the aspects of the invention, arfolitixorin (6R-MTHF) is employed as a solid form which is soluble in water, such as a lyophilizate or a salt, optionally stabilized by one or more suitable excipients and/or antioxidants such as citric acid or ascorbic acid or salt forms thereof.

In other preferred embodiments of any of the aspects of the invention the lyophilizate of arfolitixorin is reconstituted in aqueous media.

In other preferred embodiments of any of the aspects of the invention the lyophilizate of arfolitixorin is prepared from 6R-MTHF hemisulfate salt.

In other preferred embodiments of any of the aspects of the invention the lyophilizate is prepared from 6R-MTHF hemisulfate salt and trisodium citrate dihydrate.

### EXAMPLES

Arfolitixorin (former Modufolin^{®}) is a folate-based biomodulator developed by applicant to improve the outcome of a range of antimetabolite treatments used within oncology. One of the therapeutic areas of specific interest included in the development program of arfolitixorin is as biomodulator of 5-fluorouracil (5-FU) activity in standard treatment regime for advanced, metastatic CRC, such as Stage IV. A stable lyophilizate formulation of the naturally occurring diastereoisomeric folate [6R]-5,10-MTHF has successfully been made from the drug substance arfolitixorin hemisulfate. The drug substance arfolitixorin hemisulfate in itself was found to be an unexpectedly stable salt of the otherwise very unstable naturally occurring [6R]-5,10-MTHF. As mentioned in the background section of the present application, [6R]-5,10-methylenetetrahydrofolate, abbreviated herein as [6R]-5,10-MTHF, needs to be metabolically formed when using the widely used folate-based drugs leucovorin and levoleucovorin. Arfolitixorin, however, does not require metabolic activation to exert its effect and may be directly involved in the formation of the FdUMP TS ternary complex discussed hereinabove.

The below **Table 1** shows the treatment protocol for the employed chemotherapy agents (oxaliplatin, irinotecan, 5-fluorouracil, arfolitixorin and pyridoxine) employed for patients with RAS-mutated pancreatic adenocarcinoma:

**Table 1**

| | **Drug** | **Planned dose** | | | **Mode** |
|---|---|---|---|---|---|
| | | **1st level** | **2nd level** | **3rd level** | |
| **Day 1** | **oxaliplatin ^{a}** | **80 mg/m²** | **n/a** | **n/a** | **iv 120 min** |
| | **irinotecan ^{b}** | **180 mg/m²** | **n/a** | **n/a** | **iv 30 min** |
| | **[6R]-MTHF (No1) ^{c}** | **120-200 mg/m²** | **300 mg/m²** | **400 mg/m²** | **iv 30 min** |
| | **pyridoxine (No1) ^{d}** | **3000 mg** | **4500 mg** | **6000 mg** | **iv 30 min** |
| | **5-FU (No1) ^{b}** | **625 mg/m²** | **n/a** | **n/a** | **iv 120 min** |
| | **5-FU (No2) ^{b}** | **375 mg/m²** | **n/a** | **n/a** | **iv 22 hrs** |

| | **Drug** | **Planned dose** | | | **Mode** |
|---|---|---|---|---|---|
| | | **1st level** | **2nd level** | **3rd level** | |
| **Day 2** | **[6R]-MTHF (No 2) ^{c}** | **120-200 mg/m²** | **300 mg/m²** | **400 mg/m²** | **iv 30 min** |
| | **pyridoxine (No 2) ^{d}** | **3000 mg** | **4500 mg** | **6000 mg** | **iv 30 min** |
| | **5-FU (No3) ^{b}** | **625 mg/m²** | **n/a** | **n/a** | **iv 120 min** |
| | **5-FU (No4) ^{b}** | **375 mg/m²** | **n/a** | **n/a** | **iv 22 hrs** |

| | | | | | |
|---|---|---|---|---|---|
| ^{a} Doses of oxaliplatin and irinotecan are adjusted according to tolerance. The dose of oxaliplatin is decreased by 20% in case of Grade 1 SPN, and the drug is suspended in case of ≥ Grade 2 SPN ^{b} According to tolerance in previous courses, the daily dose of 5-FU can be increased by 10% increments from one course to the next (*i.e.*, C1: 80%; C2: 90%; C3: 100%; C4: 110%; C5:120% as maximum) ^{c,d} Increments from one dose level to next (e.g. 120-200 → 300 → 400 mg/m²) are performed in successive groups of 5 patients in absence of toxicity. | | | | | |

### TREATMENT AND MATERIALS

**Arfolitixorin** ([6R]-5,10-Methylene-tetrahydrofolic acid, [6R]-MTHF) is formulated as a lyophilized powder containing 100 mg per vial (calculated as free acid). Dosing: injections at a fixed dose of 120-200, 300 or 400 mg/m² are administered over 30 min after administration of 5-FU (see below) on both Day 1 and Day 2 in each treatment cycle in all cycles of the study. For the first cycle 200 mg/m² is given followed by 300 mg/m² and finally a dose of maximum 400 mg/m² is given for all subsequent cycles. The regimen will be repeated every second week for at least eight (8) cycles, i.e. sixteen (16) weeks.

**5-FU (5-fluorouracil)** is formulated as injection solution. Dosing: 5-FU is administered as infusions on Day 1 and Day 2 in each treatment cycle after injection of pyridoxine (see below). 5-FU will be administered at a fixed dose of 625 mg/m² over 120 min after finalizing the injection of pyridoxine, and again administered as an infusion of 375 mg/m² over 22 hrs The treatment will be repeated every second week for at least eight (8) cycles, i.e. sixteen (16) weeks.

**Oxaliplatin** is formulated as a concentrated infusion solution. Dosing: Oxaliplatin will be administered as an i.v. infusion of 80 mg/m² over 120 minutes on Day 1 in each treatment cycle after cetuximab/bevacizumab administration (see below), and repeated every second week for at least eight (8) cycles, i.e. sixteen (16) weeks. For the first cycle, 80% of the dose (64 mg/m²) is given before increasing to 100% (80 mg/m²) for subsequent cycles. Caution will be taken regarding toxicity associated with administration that may affect rate of infusion (e.g. grade ≤2 allergy, laryngopharyngeal dysesthesias, and laryngeal spasm). In such cases, rate of oxaliplatin administration should be prolonged in following cycles according to clinical practice recommendations.

**Irinotecan** is formulated as a concentrated infusion solution. Dosing: Irinotecan will be administered as an i.v. infusion of 180 mg/m² over 30 minutes on Day 1 in each treatment cycle after oxaliplatin administration and repeated every second week for at least eight (8) cycles, i.e. sixteen (16) weeks. For the first cycle, 80% of the dose (144 mg/m²) is given before increasing to 100% (180 mg/m²) for subsequent cycles. Caution will be taken regarding early toxicity (within 24 hours) associated with irinotecan administration, i.e. acute cholinergic syndrome, characterized by early diarrhoea, emesis, diaphoresis, abdominal cramping, and, less commonly, hyperlacrimation and rhinorrhoea. In such cases, the use of anticholinergics according to clinical practice recommendations is necessary.

**Pyridoxine** hydrochloride is a colorless or white crystal or a white crystalline powder. One gram dissolves in 5 mL of water. It is stable in air and is slowly affected by sunlight. Pyridoxine Hydrochloride Injection, USP is formulated in 5 ml vials of 250 mg pyridoxine hydrochloride in water (50 mg/ml).

Dosing: pyridoxine is administered right after arfolitixorin administration as an i.v. infusion of 3000 - 6000 mg/m² during 30 minutes on both Day 1 and Day 2 in each treatment cycle and repeated every second week for at least eight (8) cycles, i.e. sixteen (16) weeks. For the first cycle 3000 mg/m² is given followed by 4500 mg/m² and finally a dose of maximum 6000 mg/m² is given for all subsequent cycles.

Assessment of antitumor efficacy for the above dosage protocol is performed after each phase of 4 courses (i.e., before the 5th course, before the 9th course, etc.). The following variables are assessed: Antitumor response rate (according to RECIST and PERCIST); Magnitude of response; Rapidity of response (tumor shrinkage); Pathologic responses when indicated; PFS/DFS; and assessment of toxicity.

### REFERENCES

1. Simon R. Optimal two-stage designs for phase II clinical trials. Control Clin Trials. 1989;10(1):1-10. doi:10.1016/0197-2456(89)90015-9
2. Puckett Y, Garfield K. Cancer, Pancreas. StatPearls Publishing; 2018. http://www.ncbi.nlm.nih.gov/pubmed/30085538. Accessed January 21, 2021.
3. Rawla P, Sunkara T, Gaduputi V. Epidemiology of Pancreatic Cancer: Global Trends, Etiology and Risk Factors. World J Oncol. 2019;10(1):10-27. doi:10.14740/wjon1166
4. Siegel RL, Miller KD, Fuchs HE, Jemal A. Cancer Statistics, 2021. CA Cancer J Clin. 2021;71(1):7-33. doi:10.3322/caac.21654
5. American Cancer Society. American Cancer Society. Cancer Facts & Figures. 2021.
6. Ducreux M, Cuhna AS, Caramella C, et al. Cancer of the pancreas: ESMO Clinical Practice Guidelines for diagnosis, treatment and follow-up. Ann Oncol. 2015;26:v56-v68. doi:10.1093/annonc/mdv295
7. Conroy T, Desseigne F, Ychou M, et al. FOLFIRINOX versus Gemcitabine for Metastatic Pancreatic Cancer. N Engl J Med. 2011;364(19):1817-1825. doi:10.1056/nejmoa1011923
8. Von Hoff DD, Ervin T, Arena FP, et al. Increased Survival in Pancreatic Cancer with nab-Paclitaxel plus Gemcitabine. N Engl J Med. 2013;369(18):1691-1703. doi:10.1056/nejmoa1304369
9. Tempero MA, Malafa MP, Chiorean EG, et al. Pancreatic adenocarcinoma, version 1.2019 featured updates to the NCCN guidelines. JNCCN J Natl Compr Cancer Netw. 2019;17(3):203-210. doi:10.6004/jnccn.2019.0014
10. Wang-Gillam A, Li CP, Bodoky G, et al. Nanoliposomal irinotecan with fluorouracil and folinic acid in metastatic pancreatic cancer after previous gemcitabine-based therapy (NAPOLI-1): A global, randomised, open-label, phase 3 trial. Lancet. 2016;387(10018):545-557. doi:10.1016/S0140-6736(15)00986-1
11. Lowery MA, Yu KH, Adel NG, et al. Activity of front-line FOLFIRINOX (FFX) in stage III/IV pancreatic adenocarcinoma (PC) at Memorial Sloan-Kettering Cancer Center (MSKCC). J Clin Oncol. 2012;30(15_suppl):4057-4057. doi:10.1200/jco.2012.30.15_suppl.4057
12. Stein SM, James ES, Deng Y, et al. Final analysis of a phase II study of modified FOLFIRINOX in locally advanced and metastatic pancreatic cancer. Br J Cancer. 2016;114(7):737-743. doi:10.1038/bjc.2016.45
13. Ko AH, LoConte N, Tempero MA, et al. A phase I study of FOLFIRINOX Plus IPI-926, a hedgehog pathway inhibitor, for advanced pancreatic adenocarcinoma. Pancreas. 2016;45(3):370-375. doi:10.1097/MPA.0000000000000458
14. Philip PA, Buyse ME, Alistar AT, et al. Avenger 500, a phase III open-label randomized trial of the combination of CPI-613 with modified FOLFIRINOX (mFFX) versus FOLFIRINOX (FFX) in patients with metastatic adenocarcinoma of the pancreas. J Clin Oncol. 2019;37(4_suppl):TPS479-TPS479. doi:10.1200/jco.2019.37.4_suppl.tps479
15. Conroy T, Hammel P, Hebbar M, et al. FOLFIRINOX or Gemcitabine as Adjuvant Therapy for Pancreatic Cancer. N Engl J Med. 2018;379(25):2395-2406. doi:10.1056/NEJMoa1809775
16. Danenberg P V., Gustavsson B, Johnston P, et al. Folates as adjuvants to anticancer agents: Chemical rationale and mechanism of action. Crit Rev Oncol Hematol. 2016;106:118-131. doi:10.1016/j.critrevonc.2016.08.001
17. Saif MW, Merritt J, Robbins J, Stewart J, Schupp J. Phase III multicenter randomized clinical trial to evaluate the safety and efficacy of CoFactor®/5-fluorouracil/bevacizumab versus leucovorin/5-fluorouracil/bevacizumab as initial treatment for metastatic colorectal carcinoma. Clin Colorectal Cancer. 2006;6(3):229-234. doi:10.3816/CCC.2006.n.042
18. Danenberg PV, Malli H SS. Thymidylate synthase inhibitors. Semin Oncol. 1999;26(6):621-631.
19. Wettergren Y, Taflin H, Odin E, Kodeda K, Derwinger K. A pharmacokinetic and pharmacodynamic investigation of Modufolin® compared to Isovorin® after single dose intravenous administration to patients with colon cancer: a randomized study. Cancer Chemother Pharmacol. 2015;75(1):37-47. doi:10.1007/s00280-014-2611-9
20. D Machover et al. Enhancement of 5-fluorouracil cytotoxicity by pyridoxal 5'-phosphate and folinic acid in tandem. J. Pharmacol. Exp. Ther. 366, 238-243 (2018).
21. D Machover et al. Pharmacologic modulation of 5-fluorouracil by folinic acid and pyridoxine for treatment of patients with advanced breast carcinoma. Scientific Reports | (2022) 12:9079
22. D Machover et al. Pharmacologic modulation of 5-fluorouracil by folinic acid and high-dose pyridoxine for treatment of patients with digestive tract carcinomas. Scientific Reports | (2021) 11:12668
23. Hang et al. Prediction of overall survival for metastatic pancreatic cancer: Development and validation of a prognostic nomogram with data from open clinical trial and real-world study. Cancer Medicine. 2018;7:2974-2984.

## Claims

1. Arfolitixorin for use in a human patient in the treatment of pancreatic adenocarcinoma, which treatment comprises the following steps:
**On Day 1**
a) administering a continuous IV infusion over 120 min ± 5 min containing 80 mg/m² oxaliplatin, followed by
b) administering a continuous IV infusion over 30 min ± 3 min containing 180 mg/m² irinotecan, followed by
c) administering a continuous IV infusion over 30 min ± 3 min containing 150 - 400 mg/m² arfolitixorin, followed by
d) administering an IV bolus over 30 min ± 3 min containing 3000 - 6000 mg pyridoxine, followed by
e) administering a continuous IV infusion over 120 min ± 10 min containing 625 mg/m² 5-fluorouracil (5-FU), followed by
f) administering a continuous IV infusion containing 375 mg/m² 5-fluorouracil over 22 hours ± 1 hour, followed by
**On Day 2**
g) administering a continuous IV infusion over 30 min ± 3 min containing 150 - 400 mg/m² arfolitixorin, followed by
h) administering an IV bolus over 30 min ± 3 min containing 3000 - 6000 mg pyridoxine, followed by
i) administering a continuous IV infusion over 120 min ± 10 min containing 625 mg/m² 5-FU, followed by
j) administering a continuous IV infusion containing 375 mg/m² 5-FU over 22 hours ± 1 hour,
wherein said patient has been found by genotype testing to have either Ras-WT or Ras-mutated pancreatic adenocarcinoma, and wherein all steps a) - j) are repeated every 2 weeks until termination of the treatment.

2. [6R]-5,10-methylenetetrahydrofolate for use in a human patient according to claim 1, wherein said patient has Ras-WT pancreatic adenocarcinoma.

3. Arfolitixorin for use in a human patient according to claim 1, wherein said patient has Ras-mutated pancreatic adenocarcinoma.

4. Arfolitixorin for use in a human patient according to claim 3, wherein genotype testing has shown that the patient is KRAS mutation-positive.

5. Arfolitixorin for use in a human patient according to claim 1, wherein said patient has *KRAS*-mutated pancreatic adenocarcinoma.

6. Arfolitixorin for use in a human patient according to any one of claim 1 - 4, wherein genotype testing is performed by dPCR analysis of tumor tissue.

7. Arfolitixorin for use in a human patient according to any one of claim 1 - 5, wherein said patient is previously untreated for pancreatic adenocarcinoma.

8. Arfolitixorin for use in a human patient according to claim 1 - 6, wherein steps a) - j) are repeated every 2 weeks for a total treatment period of at least 16 weeks.

9. Arfolitixorin for use in a human patient according to claim 1 - 7, wherein steps a) - j) are repeated every 2 weeks for a total treatment period of at least 20 weeks, such as least 24 weeks.

10. Arfolitixorin for use in a human patient according to any one of the preceding claims wherein arfolitixorin is employed as a solid form which is soluble in water, such as a lyophilizate or a salt, optionally stabilized by one or more suitable excipients and/or antioxidants such as citric acid or ascorbic acid or salt forms thereof.

11. Arfolitixorin for use in a human patient according to any one of the preceding claims wherein 5-fluorouracil (5-FU) is replaced by a fluorinated pyrimidine base such as cape-citabine (Xeloda), i.e. N4-pentyloxycarbonyl-5'-deoxy-5-fluorocytidine, tegafur, 5-fluoro-pyrimidinone, UFT, doxifluridine, 2'-deoxy-5 fluorouridine, 5'-deoxy-5-fluorouridine, 1-(2'-oxopropyl)-5-FU, and alkyl-carbonyl-5-FU, BOF-A2, ftorafur(TS-1), and S-1.

12. Arfolitixorin for use in a human patient according to any one of the preceding claims wherein treatment is terminated by a patient decision or a decision taken by the responsible medical person, i.a. due to disease progression, adverse events or treatment holidays.
